(19) Europäisches Patentamt
European Patent Office
Office européen des brevets

(11) EP 4 475 251 B1

(12) EUROPEAN PATENT SPECIFICATION

(45) Date of publication and mention of the grant of the patent:
**01.10.2025 Bulletin 2025/40**

(21) Application number: **24180097.8**

(22) Date of filing: **05.06.2024**

(51) International Patent Classification (IPC):
***H01M 10/052*** *(2010.01)* ***H01M 10/0525*** *(2010.01)*
***H01M 10/0567*** *(2010.01)* ***H01M 10/42*** *(2006.01)*

(52) Cooperative Patent Classification (CPC):
**H01M 10/0567; H01M 10/052; H01M 10/0525; H01M 10/4235;** H01M 2300/0025; Y02E 60/10

# (54) ELECTROLYTE FOR RECHARGEABLE LITHIUM BATTERY AND RECHARGEABLE LITHIUM BATTERY INCLUDING THE SAME

ELEKTROLYT FÜR EINE WIEDERAUFLADBARE LITHIUMBATTERIE UND WIEDERAUFLADBARE LITHIUMBATTERIE DAMIT

ÉLECTROLYTE POUR BATTERIE AU LITHIUM RECHARGEABLE ET BATTERIE AU LITHIUM RECHARGEABLE LE COMPRENANT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.06.2023 KR 20230073678**

(43) Date of publication of application:
**11.12.2024 Bulletin 2024/50**

(73) Proprietor: **Samsung SDI Co., Ltd.**
**Yongin-si, Gyeonggi-do 17084 (KR)**

(72) Inventors:
- **Lee, Tae Jin**
**Yongin-si, Gyeonggi-do 17084 (KR)**
- **Cha, Si-Young**
**Yongin-si, Gyeonggi-do 17084 (KR)**
- **Kang, Inah**
**Yongin-si, Gyeonggi-do 17084 (KR)**
- **Sim, Minji**
**Yongin-si, Gyeonggi-do 17084 (KR)**
- **Jeong, Hanmam**
**Yongin-si, Gyeonggi-do 17084 (KR)**

(74) Representative: **Gulde & Partner**
**Patent- und Rechtsanwaltskanzlei mbB**
**Berliner Freiheit 2**
**10785 Berlin (DE)**

(56) References cited:
**WO-A1-2022/150849**
**JP-A- 2017 228 520**
**US-A1- 2012 088 160**
**US-A1- 2014 308 564**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 475 251 B1

## Description

## BACKGROUND

### 1. Field

According to one or more embodiments, the present disclosure relates to an electrolyte for a rechargeable lithium battery and a rechargeable lithium battery including the same.

### 2. Description of the Related Art

A rechargeable lithium battery may be recharged and has three or more times the (higher)energy density per unit weight as compared to a comparable lead storage battery, nickel-cadmium battery, nickel hydrogen battery, nickel zinc battery and/or the like. The rechargeable lithium battery may be also charged at a relatively high rate and thus, is commercially manufactured for a laptop, a cell phone, an electric tool, an electric bike, and/or the like, and researches on improvement of additional energy density have been actively made or pursued.

Such a rechargeable lithium battery is manufactured by injecting an electrolyte into an electrode assembly, which includes a positive electrode (e.g., including a positive electrode active material) and a negative electrode (e.g., including a negative electrode active material).

Recently, one developmental advance in the technology of rechargeable lithium batteries is to improve relatively high-temperature performance characteristics. In general, the rechargeable lithium batteries may experience an increase in electrical resistance at a relatively high temperature, that may increase the likelihood of an ignition and/or explosion. For example, in a module and/or a pack assembly including several rechargeable lithium battery cells, excess heat produced in one of the rechargeable lithium battery cells may spread to the adjacent cells in succession, thereby causing the entire module and/or pack to overheat. The patents US2014/308564A1, US2012/088160A1, WO2022/150849A1, and JP2017228520A all disclose electrolytes comprising a first additive according to Chemical Formula 1 of the present invention.

## SUMMARY

The invention is defined by the appended claims. The description that follows is subjected to this limitation. Any disclosure lying outside the scope of said claims is only intended for illustrative as well as comparative purposes.

One or more aspects are directed toward an electrolyte for a rechargeable lithium battery that may suppress or reduce ignition and/or explosion of the corresponding battery cell at relatively high temperatures. In some aspects, the electrolyte may prevent or reduce a temperature increase in the corresponding battery cell even if (e.g., when) an ignition and/or explosion occurs, or begins to occur, in an adjacent battery cell.

Additional aspects will be set forth in part in the description which follows and, in part, will be apparent from the description, or may be learned by practice of the presented embodiments of the disclosure.

The rechargeable lithium battery of one or more embodiments is capable of suppressing or reducing ignition and/or explosion of the corresponding battery cell at relatively high temperatures by combining the two types (kinds) of additives disclosed herein. In some embodiments, the rechargeable lithium battery prevents the temperature of the corresponding battery cell from (protect from temperature) increasing even if (e.g. when) an ignition and/or explosion occurs, or begins to occur, in an adjacent battery cell.

## BRIEF DESCRIPTION OF THE DRAWING

The drawing is a schematic view showing a rechargeable lithium battery according to one or more embodiments.

## DETAILED DESCRIPTION

Hereinafter, a rechargeable lithium battery according to one or more embodiments will be described in more detail with reference to the accompanying drawing, so that those of ordinary skill in the art can easily implement them. Examples of the embodiments are illustrated and described by referring to the accompanying drawing to explain aspects of the present description. However, these embodiments are merely examples, and the present disclosure is not limited thereto. Rather the present disclosure may be embodied in many different forms and is defined by the scope of claims.

The terminology utilized herein is utilized to describe embodiments only, and is not intended to limit the present disclosure. The singular expressions "a," "an," and "the" include the plural expressions, including "at least one," unless the context clearly dictates otherwise.

Herein, it should be understood that terms such as "comprises," "comprise," "comprising," "includes," "including," "include," "having," "has," and/or "have" are intended to designate the presence of an embodied aspect, number, operation, element, and/or any suitable combination thereof, but it does not preclude the possibility of the presence or addition of one or more other feature, number, operation, element, and/or any suitable combination thereof.

In the drawing, the thickness of layers, films, panels, regions, and/or the like, are exaggerated for clarity wherein like reference numerals designate like elements, and duplicative descriptions thereof may not be provided throughout the specification. It will be understood that if (e.g., when) an element such as a layer, film, region, or substrate is referred to as being "on" another element, it can be directly on the other element or intervening elements may also be present. In contrast, if (e.g., when) an element is referred to as being "directly on" another element, there are no intervening elements present.

In one or more embodiments, the term "layer" herein includes not only a shape formed on the whole surface if (e.g., when) viewed from a plan view, but also a shape formed on a partial surface.

It will be understood that, although the terms "first," "second," "third," and/or the like may be utilized herein to describe one or more suitable elements, components, regions, layers and/or sections, these elements, components, regions, layers and/or sections should not be limited by these terms. These terms are only utilized to distinguish one element, component, region, layer or section from another element, component, region, layer, or section. Thus, a first element, component, region, layer, or section described herein may be termed a second element, component, region, layer or section without departing from the teachings set forth herein.

Spatially relative terms, such as "beneath," "below," "lower," "above," "upper" and/or the like, may be utilized herein to easily describe the relationship between one element or feature and another element or feature. It will be understood that the spatially relative terms are intended to encompass different orientations of a device in utilization or operation in addition to the orientation illustrated in the drawings. For example, if (e.g., when) the device in the drawing is turned over, elements described as "below" or "beneath" other elements or features will be oriented "above" the other elements or features. Thus, the example term "below" can encompass both (e.g., simultaneously) the orientations of above and below. The device may be otherwise oriented (rotated 90 degrees or at other orientations), and the spatially relative terms utilized herein may be interpreted accordingly.

Unless otherwise defined, all terms (including chemical, technical and scientific terms) utilized herein have the same meaning as commonly understood by one of ordinary skill in the art to which this disclosure pertains. It will be further understood that terms, such as those defined in commonly utilized dictionaries, should be interpreted as having a meaning that is consistent with their meaning in the context of the related art and the present disclosure, and will not be interpreted in an idealized or overly formal sense.

Example embodiments are described herein with reference to a schematic view of idealized embodiments. As such, variations from the shapes of the illustrations as a result, for example, of manufacturing techniques and/or tolerances, are to be expected. Thus, embodiments described herein should not be construed as being limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the drawing are schematic in nature and their shapes are not intended to illustrate the precise shape of a region and are not intended to limit the scope of the present claims.

The term "may" will be understood to refer to "one or more embodiments of the present disclosure," some of which include the described element and some of which exclude that element and/or include an alternate element. Similarly, alternative language such as "or" refers to "one or more embodiments of the present disclosure," each including a corresponding listed item.

In this context, "consisting essentially of" means that any additional components will not materially affect the chemical, physical, optical or electrical properties of the semiconductor film.

Further, in this specification, the phrase "on a plane," or "plan view," means viewing a target portion from the top, and the phrase "on a cross-section" means viewing a cross-section formed by vertically cutting a target portion from the side.

## Definitions

The term "particle diameter" as utilized herein refers to an average diameter of particles if (e.g., when) the particles are spherical, and refers to an average major axis length of particles if (e.g., when) the particles are non-spherical. For example, the average particle diameter may be measured by a method well suitable to those skilled in the art, for example, may be measured by a particle size analyzer, or may be measured by a transmission electron microscopic image or a scanning electron microscopic image. It may be possible to obtain an average particle diameter value by measuring utilizing a dynamic light scattering method, performing data analysis, counting the number of particles for each particle size range, and calculating from this. Unless otherwise defined, the average particle diameter may refer to the diameter ($D_{50}$) of

particles having a cumulative volume of 50 volume% in the particle size distribution. If (e.g., when) measuring by laser diffraction, more specifically, the particles to be measured are dispersed in a dispersion medium and then introduced into a related art laser diffraction particle size measuring device (e.g., MT 3000 available from Microtrac, Ltd.) utilizing ultrasonic waves at about 28 kHz, and after irradiation with an output of 60 W, the average particle diameter (D50) based on 50% of the particle size distribution in the measuring device can be calculated. As utilized herein, if (e.g., when) a definition is not otherwise provided, the average particle diameter refers to a diameter ($D_{50}$) of particles having a cumulative volume of 50 volume% in the particle size distribution that is obtained by measuring the size (diameter or length of the major axis) of about 20 particles at random in a scanning electron microscopic image.

In present disclosure, "not include a or any 'component'" "exclude a or any 'component'", "'component'-free", and/or the like refers to that the "component" not being added, selected or utilized as a component in the composition/-structure, but the "component" of less than a suitable amount may still be included due to other impurities and/or external factors.

"Metal" is interpreted as a concept including ordinary metals, transition metals and metalloids (semi-metals).

As utilized herein, if (e.g., when) specific definition is not otherwise provided, "substituted" refers to replacement of at least one hydrogen of a compound by a halogen atom (F, Cl, Br, or I), a hydroxy group, a C1 to C20 alkoxy group, a nitro group, a cyano group, an amine group, an imino group, an azido group, an amidino group, a hydrazino group, a hydrazono group, a carbonyl group, a carbamyl group, a thiol group, an ester group, an ether group, a carboxyl group or a salt thereof, a sulfonic acid group or a salt thereof, a phosphoric acid or a salt thereof, a C1 to C20 alkyl group, a C2 to C20 alkenyl group, a C2 to C20 alkynyl group, a C6 to C30 aryl group, a C3 to C20 cycloalkyl group, a C3 to C20 cycloalkenyl group, a C3 to C20 cycloalkynyl group, a C2 to C20 heterocycloalkyl group, a C2 to C20 heterocycloalkenyl group, a C2 to C20 heterocycloalkynyl group, and/or a (e.g., any suitable) combination thereof.

As utilized herein, if (e.g., when) specific definition is not otherwise provided, "heterocycloalkyl group", "heterocycloalkenyl group", "heterocycloalkynyl group," and "heterocycloalkylene group" refer to presence of at least one N, O, S, or P in a cyclic compound of cycloalkyl, cycloalkenyl, cycloalkynyl, and cycloalkylene.

In the chemical formula of the present specification, unless a specific definition is otherwise provided, hydrogen is bonded at the position if (e.g., when) a chemical bond is not drawn where supposed to be given.

As utilized herein, if (e.g., when) a definition is not otherwise provided, " * " refers to a linking part between the same or different atoms, or chemical formulas.

**Electrolyte**

An electrolyte for a rechargeable lithium battery includes a non-aqueous organic solvent; a lithium salt; a first additive represented by Chemical Formula 1; and a second additive represented by Chemical Formula 2:

Chemical Formula 1

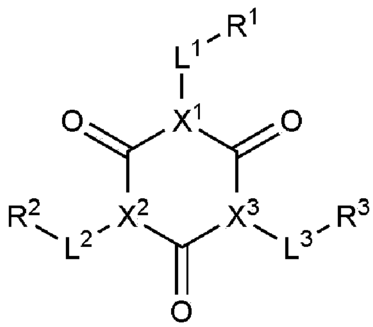

Chemical Formula 2

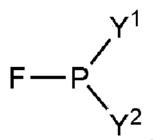

The first additive has the aspect of gelling the electrolyte at relatively high temperatures. The second additive has the aspect of delaying structural collapse of the positive electrode surface at relatively high temperatures by binding to the

positive electrode transition metal oxide (e.g., as a ligand).

Overall, the combination of the two types (kinds) of additives described herein has the aspect of rapidly increasing the viscosity of the electrolyte (e.g., at relatively high temperatures) and, at the same time, drastically reducing ionic conductivity. For example, the combined properties may thereby disconnect (e.g., shut down) the rechargeable lithium battery cell (e.g., from the surroundings, such as other battery cells).

Accordingly, a rechargeable lithium battery in which the electrolyte for a rechargeable lithium battery of one or more embodiments is utilized or applied can suppress or reduce ignition and/or explosion of the cell at relatively high temperature by a combination of the two types (kinds) of additives described herein, and even if (e.g., when) ignition and/or explosion starts in an adjacent cell, the temperature increase in that cell can be prevented or reduced.

Hereinafter, an electrolyte for a rechargeable lithium battery of one or more embodiments will be described in more detail.

**First Additive**

The description of Chemical Formula 1 representing the first additive is as follows.

$X^1$ to $X^3$ are each N or CH, provided that at least one of (e.g., selected from among) $X^1$ to $X^3$ is N.

In one or more embodiments, $X^1$ to $X^3$ may each (e.g., all) be N.

$L^1$ to $L^3$ are each independently a single bond, or a substituted or unsubstituted C1 to C10 alkylene group.

In one or more embodiments, $L^1$ to $L^3$ may each independently be a substituted or unsubstituted C1 to C5 alkylene group.

$R^1$ to $R^3$ are each independently an epoxy group, or a substituted or unsubstituted C1 to C10 alkyl group, provided that at least one of (e.g., selected from among) $R^1$ to $R^3$ is epoxy group.

In one or more embodiments, $R^1$ to $R^3$ may each (e.g., all) be an epoxy group. In one or more embodiments, the first additive (e.g., Chemical Formula 1) may be represented by Chemical Formula 1-1:

Chemical Formula 1-1

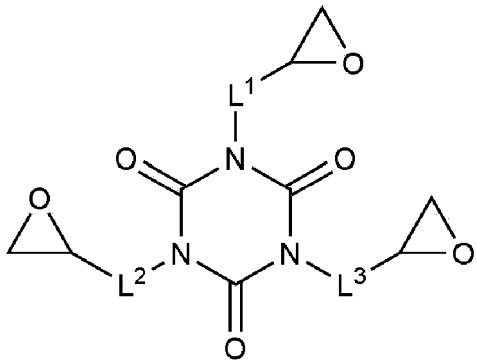

In Chemical Formula 1-1,
$L^1$ to $L^3$ may each independently be a substituted or unsubstituted C1 to C5 alkylene group.

Representative examples of the first additive are as follows:

Chemical Formula 1-1-1

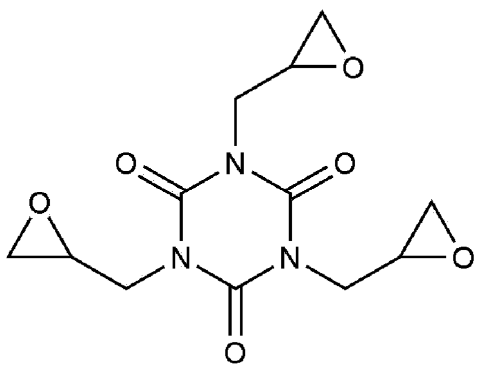

Chemical Formula 1-1-2

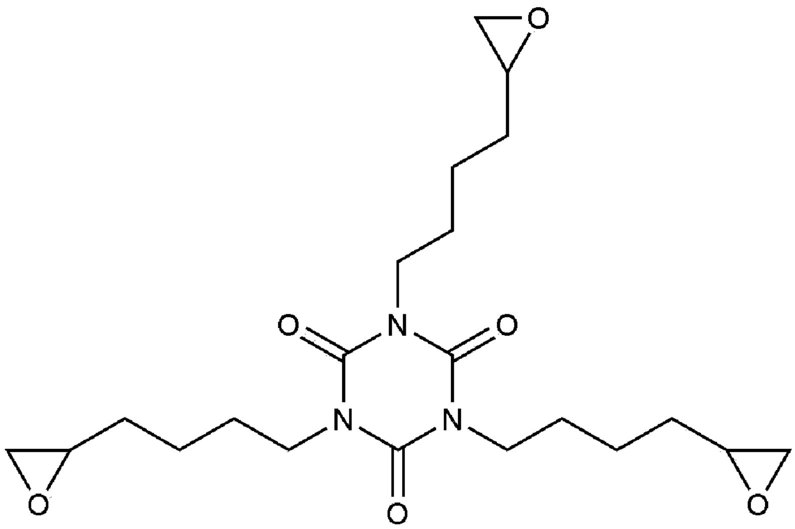

.

**Second Additive**

The description of Chemical Formula 2 representing the second additive is as follows.

$Y^1$ and $Y^2$ are each independently a halogen group or $-O-L^4-R^4$, provided that at least one of (e.g., selected from among) $Y^1$ and $Y^2$ is $-O-L^4-R^4$.

$L^4$ is a single bond or a substituted or unsubstituted C1 to C10 alkylene group.

$R^4$ is a cyano group (-CN), a difluorophosphite group ($-OPF_2$), a substituted or unsubstituted C1 to C10 alkyl group, a substituted or unsubstituted C2 to C10 alkenyl group, a substituted or unsubstituted C3 to C10 cycloalkyl group, a substituted or unsubstituted C3 to C10 cycloalkenyl group, a substituted or unsubstituted C2 to C10 alkynyl group, a substituted or unsubstituted C3 to C10 cycloalkynyl group, or a substituted or unsubstituted C6 to C20 aryl group.

If (e.g., when) $Y^1$ and $Y^2$ are concurrently (e.g., simultaneously) $-O-L^4-R^4$, two $R^4$s may not be linked (e.g., exists independently) or two $R^4$s may be linked to form (or provide) a substituted or unsubstituted monocyclic or polycyclic aliphatic heterocycle, or a substituted or unsubstituted monocyclic or polycyclic aromatic heterocycle.

In one or more embodiments, one selected from among (e.g., either) $Y^1$ and $Y^2$ may be a fluoro atom and the other one (e.g., one selected from among $Y^1$ and $Y^2$), may be $-O-L^5-R^5$; wherein $L^5$ may be a single bond or a substituted or unsubstituted C1 to C10 alkylene group; and $R^5$ may be a cyano group (-CN) or a difluorophosphite group ($-OPF_2$).

In one or more embodiments, $Y^1$ may be $-O-L^6-R^6$ and $Y^2$ may be $O-L^7-R^7$; wherein $L^6$ and $L^7$ may each independently be a single bond or a substituted or unsubstituted C1 to C10 alkylene group; $R^6$ and $R^7$ may each independently be a substituted or unsubstituted C1 to C10 alkyl group, and $R^6$ and $R^7$ may be linked to form (or provide) a substituted or unsubstituted monocyclic or polycyclic aliphatic heterocycle.

In one or more embodiments, the second additive (e.g., Chemical Formula 2) may be represented by Chemical Formula 2-1 or 2-2:

Chemical Formula 2-1

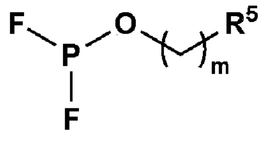

wherein, in Chemical Formula 1-1,

m may be an integer of 1 to 5; and
$R^5$ may be a cyano group (-CN) or a difluorophosphite group ($-OPF_2$).

Chemical Formula 2-2

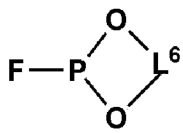

wherein, in Chemical Formula 1-2,

$L^6$ may be a substituted or unsubstituted C2 to C5 alkylene group.

In one or more embodiments, the second additive (e.g., Chemical Formula 2-2) may be represented by Chemical Formula 2-2a or 2-2b:

Chemical Formula 2-2a

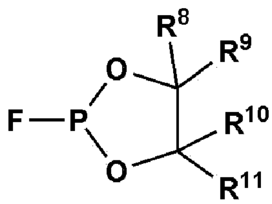

Chemical Formula 2-2b

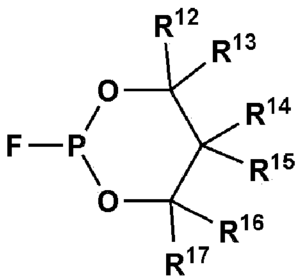

wherein, in Chemical Formulas 2-2a and 2-2b,

$R^8$ to $R^{17}$ may each independently be a hydrogen atom, a halogen atom, or a substituted or unsubstituted C1 to C5 alkyl group.

Representative examples of the second additive are as follows:

Chemical Formula 2-1a-1

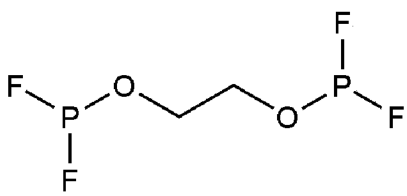

Chemical Formula 2-1a-2

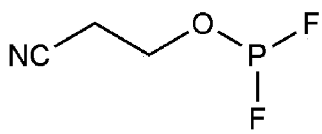

Chemical Formula 2-2a-1

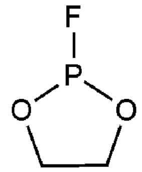

Chemical Formula 2-2a-2

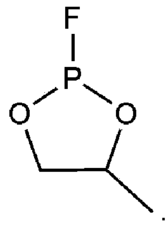

**Contents of First Additive and Second Additive**

In one or more embodiments, a weight ratio of the first additive and the second additive may be 2:1 to 100:1, or 3.5:1 to 70:1.

In one or more embodiments, a sum of the first additive and the second additive may be included in an amount of 5 to 30 wt%, or 7.1 to 21 wt% based on a total amount of the electrolyte.

In one or more embodiments, the first additive may be included in an amount of 5 to 20 wt% based on a total amount of the electrolyte. Additionally, in one or more embodiments, the second additive may be included in an amount of 0.1 to 2 wt% based on a total amount of the electrolyte.

In the disclosed ranges, there may be a synergistic aspect due to the combination of the two types (kinds) of additives described herein.

**Non-aqueous Organic Solvent**

The non-aqueous organic solvent serves as a medium for transmitting ions taking part in the electrochemical reaction of a battery.

The non-aqueous organic solvent may include a carbonate-based, ester-based, ether-based, ketone-based, alcohol-based, or aprotic solvent, and/or a (e.g., any suitable) combination thereof.

The carbonate-based solvent may include dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and/or the like. The ester-based solvent may include methyl acetate, ethyl acetate, n-propyl acetate, dimethylacetate, methylpropionate, ethyl propionate, decanolide, mevalonolactone, valerolactone, caprolactone, and/or the like. The ether-based solvent may include dibutyl ether, tetraglyme, diglyme, dimethoxyethane, 2-methyltetrahydrofuran, 2,5-dimethyltetrahydrofuran, tetrahydrofuran, and/or the like. The ketone-based solvent may include cyclohexanone and/or the like. The alcohol-based solvent may include ethanol, isopropyl alcohol, and/or the like, and the aprotic solvent may include nitriles such as R-CN (wherein R is a C2 to C20 linear, branched, or cyclic hydrocarbon group, a double bond, an aromatic ring, or an ether group), amides such as dimethylformamide, and/or the like; dioxolanes such as 1,3-dioxolane, 1,4-dioxolane, and/or the like; sulfolanes, and/or the like.

The non-aqueous organic solvent may be utilized alone or in combination with one or more (e.g., of them), and if (e.g., when) utilized in combination with one or more, a mixing ratio may be appropriately or suitably adjusted according to the desired or suitable battery performance, which is well understood by those skilled in the art.

Additionally, if (e.g., when) utilizing a carbonate-based solvent, a cyclic carbonate and a chain carbonate may be mixed and utilized. For example, the cyclic carbonate and the chain carbonate may be mixed at a volume ratio of about 1:1 to about 1:9.

The non-aqueous organic solvent may further include an aromatic hydrocarbon-based organic solvent. For example, a carbonate-based solvent and an aromatic hydrocarbon-based organic solvent may be mixed and utilized in a volume ratio of about 1:1 to about 30:1.

The electrolyte may further include vinylethyl carbonate, vinylene carbonate, or an ethylene carbonate-based compound to improve battery cycle-life.

Representative examples of the ethylene carbonate-based compound may include fluoroethylene carbonate, difluoroethylene carbonate, chloroethylene carbonate, dichloroethylene carbonate, bromoethylene carbonate, dibromoethylene carbonate, nitroethylene carbonate, cyanoethylene carbonate, and/or the like.

**Lithium Salt**

The lithium salt is dissolved in an organic solvent, supplies a battery with lithium ions, enables the general operation of the rechargeable lithium battery, and improves transportation of the lithium ions between positive and negative electrodes. Examples of the lithium salt may include at least one selected from among $LiPF_6$, $LiBF_4$, $LiSbF_6$, $LiAsF_6$, $LiClO_4$, $LiAlO_2$, $LiAlCl_4$, $LiPO_2F_2$, LiCl, LiI, $LiN(SO_3C_2F_5)_2$, $Li(FSO_2)_2N$ (lithium bis(fluorosulfonyl)imide, LiFSI), $LiC_4F_9SO_3$, $LiN(C_xF_{2x+1}SO_2)(C_yF_{2y+1}SO_2)$, x and y are integers in a range of 1 to 20, lithium trifluoromethane sulfonate, lithium tetrafluoroethane sulfonate, lithium difluorobis(oxalato)phosphate (LiDFOB), lithium bis(oxalato) borate (LiBOB).

A concentration of the lithium salt may be utilized within the range of about 0.1 M to about 2.0 M. If (e.g., when) the concentration of the lithium salt is within the described range, the electrolyte has appropriate or suitable conductivity and viscosity, and thus excellent or suitable electrolyte performance can be exhibited, and lithium ions can move effectively.

**Rechargeable Lithium Battery**

In one or more embodiments, a rechargeable lithium battery includes a positive electrode; a negative electrode; and the electrolyte according to one or more of the aforementioned embodiments.

The rechargeable lithium battery of one or more embodiments includes the additive of the aforementioned embodiment, or the electrolyte of the aforementioned embodiment, thereby suppressing or reducing ignition and/or explosion of the corresponding cell at relatively high temperatures, and preventing or reducing temperature increase in the corresponding cell even if (e.g., when) ignition and/or explosion begins in an adjacent cell.

Hereinafter, descriptions that overlap with the preceding disclosure will not be provided, and the rechargeable lithium battery will be described in more detail.

**Positive Electrode Active Material**

The positive electrode active material may be a compound capable of reversibly intercalating and deintercalating lithium (a lithiated intercalation compound). For example, one or more types (kinds) of composite oxides of lithium and a metal selected from among cobalt, manganese, nickel, and/or a (e.g., any suitable) combination thereof may be utilized.

The composite oxide may be a lithium transition metal composite oxide, and specific examples may include lithium nickel-based oxide, lithium cobalt-based oxide, lithium manganese-based oxide, lithium iron phosphate-based compound, cobalt-free lithium nickel-manganese-based oxide, and/or a (e.g., any suitable) combination thereof.

As an example, the positive electrode active material may be a relatively high nickel-based positive electrode active material having a nickel content (e.g., amount) of about 80 mol% or more based on 100 mol% of metals excluding lithium in the lithium transition metal composite oxide. The nickel content (e.g., amount) in the relatively high nickel-based positive electrode active material may be about 85 mol% or more, about 90 mol% or more, about 91 mol% or more, or about 94 mol% or more and about 99 mol% or less based on 100 mol% of metals excluding lithium. High-nickel-based positive electrode active materials can achieve relatively high capacity and can be applied to a relatively high-capacity, relatively high-density rechargeable lithium battery.

As a more specific example, a compound represented by any of the following chemical formulas may be utilized. $Li_aA_{1-b}X_bO_{2-c}D_c$ ($0.90 \leq a \leq 1.8$, $0 \leq b \leq 0.5$, $0 \leq c \leq 0.05$); $Li_aMn_{2-b}X_bO_{4-c}D_c$ ($0.90 \leq a \leq 1.8$, $0 \leq b \leq 0.5$, $0 \leq c \leq 0.05$); $Li_aNi_{1-b-c}Co_bX_cO_{2-\alpha}D_\alpha$ ($0.90 \leq a \leq 1.8$, $0 \leq b \leq 0.5$, $0 \leq c \leq 0.5$, $0 < \alpha < 2$); $Li_aNi_{1-b-c}Mn_bX_cO_{2-\alpha}D_\alpha$ ($0.90 \leq a \leq 1.8$, $0 \leq b \leq 0.5$, $0 \leq c \leq 0.5$, $0 < \alpha < 2$); $Li_aNi_bCo_cL^1_dG_eO_2$ ($0.90 \leq a \leq 1.8$, $0 \leq b \leq 0.9$, $0 \leq c \leq 0.5$, $0 \leq d \leq 0.5$, $0 \leq e \leq 0.1$); $Li_aNiG_bO_2$ ($0.90 \leq a \leq 1.8$, $0.001 \leq b \leq 0.1$); $Li_aCoG_bO_2$ ($0.90 \leq a \leq 1.8$, $0.001 \leq b \leq 0.1$); $Li_aMn_{1-b}G_bO_2$ ($0.90 \leq a \leq 1.8$, $0.001 \leq b \leq 0.1$); $Li_aMn_2G_bO_4$ ($0.90 \leq a \leq 1.8$, $0.001 \leq b \leq 0.1$); $Li_aMn_{1-g}G_gPO_4$ ($0.90 \leq a \leq 1.8$, $0 \leq g \leq 0.5$); $Li_{(3-f)}Fe_2(PO_4)_3$ ($0 \leq f \leq 2$); $Li_aFePO_4$ ($0.90 \leq a \leq 1.8$)

In the preceding chemical formulas, A is Ni, Co, Mn, and/or a (e.g., any suitable) combination thereof; X is Al, Ni, Co, Mn, Cr, Fe, Mg, Sr, V, a rare earth element, and/or a (e.g., any suitable) combination thereof; D is O, F, S, P, and/or a (e.g., any suitable) combination thereof; G is Al, Cr, Mn, Fe, Mg, La, Ce, Sr, V, and/or a (e.g., any suitable) combination thereof; Q is Ti, Mo, Mn, and/or a (e.g., any suitable) combination thereof; Z is Cr, V, Fe, Sc, Y, and/or a (e.g., any suitable) combination thereof; and $L^1$ is Mn, Al, and/or a (e.g., any suitable) combination thereof.

## Positive Electrode

The positive electrode for a rechargeable lithium battery may include a current collector and a positive electrode active material layer formed on the current collector. The positive electrode active material layer includes a positive electrode active material and may further include a binder and/or a conductive material.
A content (e.g., amount) of the positive electrode active material may be about 90 wt% to about 99.5 wt% based on 100 wt% of the positive electrode active material layer.
In some example embodiments, the positive electrode active material layer may further include a binder and a conductive material. At this time, each content (e.g., amount) of the binder and the conductive material may be about 0.5 wt% to about 5 wt% based on 100% by weight of the positive electrode active material layer.
The binder serves to ensure that the positive electrode active material particles adhere to each other and also to adhere the positive electrode active material to the current collector. Examples of the binder may include polyvinyl alcohol, carboxymethyl cellulose, hydroxypropyl cellulose, diacetyl cellulose, polyvinyl chloride, carboxylated polyvinyl chloride, polyvinyl fluoride, an ethylene oxide-containing polymer, polyvinylpyrrolidone, polyurethane, polytetrafluoroethylene, polyvinylidene fluoride, polyethylene, polypropylene, a styrene-butadiene rubber, a (meth)acrylated styrene-butadiene rubber, an epoxy resin, a (meth)acrylic resin, a polyester resin, and nylon, but are not limited thereto.
The conductive material is utilized to impart conductivity to the electrode, and in the battery being configured, any electronically conductive material can be utilized as long as it does not cause chemical change. Examples of the conductive material may include a carbon-based material such as natural graphite, artificial graphite, carbon black, acetylene black, ketjen black, a carbon fiber, a carbon nanofiber, and a carbon nanotube; a metal-based material including copper, nickel, aluminum, silver, and/or the like, and in the form of (or provide) a metal powder or a metal fiber; a conductive polymer such as a polyphenylene derivative; and/or a (e.g., any suitable) mixture thereof.
The current collector may include Al, but the present disclosure is not limited thereto.

## Negative Electrode Active Material

The negative electrode active material may be a material that reversibly intercalates/deintercalates lithium ions, a lithium metal, a lithium metal alloy, a material capable of doping and dedoping lithium, or a transition metal oxide.
The material that reversibly intercalates/deintercalates lithium ions may be a carbon-based negative electrode active material, for example crystalline carbon, amorphous carbon and/or a (e.g., any suitable) combination thereof. Examples of the crystalline carbon may include graphite such as irregular-shaped, plate-shaped, flake, spherical or fibrous natural graphite or artificial graphite, and examples of the amorphous carbon may include soft carbon or hard carbon, a mesophase pitch carbonized product, calcined coke, and/or the like.
The lithium metal alloy may include an alloy including lithium and a metal selected from among Na, K, Rb, Cs, Fr, Be, Mg, Ca, Sr, Si, Sb, Pb, In, Zn, Ba, Ra, Ge, Al, and Sn.
A Si-based negative electrode active material or a Sn-based negative electrode active material may be utilized as a material capable of doping and dedoping lithium. The Si-based negative electrode active material may include silicon, a silicon-carbon composite, SiOx ($0 < x \leq 2$), a Si-Q alloy (wherein Q is an element selected from among an alkali metal, an alkaline earth metal, a Group 13 element, Group 14 element (excluding Si), Group 15 element, Group 16 element, a transition metal, a rare earth element, and/or a (e.g., any suitable) combination thereof, for example Mg, Ca, Sr, Ba, Ra, Sc, Y, Ti, Zr, Hf, Rf, V, Nb. , Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Tl, Ge, P, As, Sb, Bi, S, Se, Te, Po, and/or a (e.g., any suitable) combination thereof), and/or a (e.g., any suitable) combination thereof. The Sn-based negative electrode active material may be Sn, $SnO_2$, $SnO_x$ ($0 < x < 2$), a Sn alloy, and/or a (e.g., any suitable) combination thereof.
The silicon-carbon composite may be a composite of silicon and amorphous carbon. An average particle diameter ($D_{50}$) of the silicon-carbon composite particles may be, for example, about 0.5 micrometer (μm) to about 20 μm. According to some example embodiments, the silicon-carbon composite may be in the form of (or provide) silicon particles and amorphous carbon coated on the surface of the silicon particles. For example, it may include a secondary particle (core) in which silicon primary particles are assembled (agglomerated) and an amorphous carbon coating layer (shell) on the surface of the secondary particle. The amorphous carbon may also be arranged between the silicon primary particles, and for example, the silicon primary particles may be coated with amorphous carbon. The secondary particles may be (exist) dispersed in an amorphous carbon matrix.
The silicon-carbon composite may further include crystalline carbon. For example, the silicon-carbon composite may include a core including crystalline carbon and silicon particles and an amorphous carbon coating layer on the surface of the core. The crystalline carbon may be artificial graphite, natural graphite, and/or a (e.g., any suitable) combination thereof. The amorphous carbon may include soft carbon or hard carbon, a mesophase pitch carbonized product, and calcined coke.
If (e.g., when) the silicon-carbon composite includes silicon and amorphous carbon, a content (e.g., amount) of

silicon may be about 10 wt% to about 50 wt%, and a content (e.g., amount) of amorphous carbon may be about 50 wt% to about 90 wt% based on 100 wt% of the silicon-carbon composite. In one or more embodiments, if (e.g., when) the composite includes silicon, amorphous carbon, and crystalline carbon, a content (e.g., amount) of silicon may be about 10 wt% to about 50 wt%, and a content (e.g., amount) of crystalline carbon may be about 10 wt% to about 70 wt%, and a content (e.g., amount) of amorphous carbon may be about 20 wt% to about 40 wt% based on 100 wt% of the silicon-carbon composite.

Additionally, a thickness of the amorphous carbon coating layer may be about 5 nanometer (nm) to about 100 nm. An average particle diameter ($D_{50}$) of the silicon particles (primary particles) may be about 10 nm to about 1 $\mu$m, or about 10 nm to about 200 nm. The silicon particles may exist as silicon alone, in the form of (or provide) a silicon alloy, or in an oxidized form. The oxidized form of silicon may be represented by $SiO_x$ ($0<x\leq2$). At this time, an atomic content (e.g., amount) ratio of Si:O, which indicates a degree of oxidation, may be about 99:1 to about 33:67. In the present specification, as utilized herein, if (e.g., when) a definition is not otherwise provided, an average particle diameter ($D_{50}$) indicates a particle where an accumulated volume is about 50 vol% in a particle size distribution.

The Si-based negative electrode active material or Sn-based negative electrode active material may be utilized by mixing with a carbon-based negative electrode active material. If (e.g., when) utilizing a mixture of Si-based negative electrode active material or Sn-based negative electrode active material and carbon-based negative electrode active material, a mixing ratio may be about 1:99 to about 90:10 by weight.

**Negative Electrode**

A negative electrode for a rechargeable lithium battery includes a current collector and a negative electrode active material layer on the current collector. The negative electrode active material layer includes a negative electrode active material and may further include a binder and/or a conductive material.

A content (e.g., amount) of the negative electrode active material may be about 95 wt% to about 99.5 wt% based on 100 wt% of the negative electrode active material layer. A content (e.g., amount) of the binder may be about 0.5 wt% to about 5 wt% based on 100 wt% of the negative electrode active material layer. A content (e.g., amount) of the binder may be about 0.5 wt% to about 5 wt% based on 100 wt% of the negative electrode active material layer. For example, the negative electrode active material layer may include about 90 wt% to about 99 wt% of the negative electrode active material, about 0.5 wt% to about 5 wt% of the binder, and about 0.5 wt% to about 5 wt% of the conductive material.

The binder serves to adhere the negative electrode active material particles to each other and also helps the negative electrode active material to adhere to the current collector. The binder may be a non-aqueous binder, an aqueous binder, a dry binder, and/or a (e.g., any suitable) combination thereof.

The non-aqueous binder may include polyvinyl chloride, carboxylated polyvinyl chloride, polyvinyl fluoride, an ethylene propylene copolymer, polystyrene, polyurethane, polytetrafluoroethylene, polyvinylidene fluoride, polyethylene, polypropylene, polyamide-imide, polyimide, and/or a (e.g., any suitable) combination thereof.

The aqueous binder may be (e.g., may be selected from among) a styrene-butadiene rubber, a (meth)acrylated styrene-butadiene rubber, a (meth)acrylonitrile-butadiene rubber, a (meth)acrylic rubber, a butyl rubber, a fluorine rubber, polyethylene oxide, polyvinylpyrrolidone, polyepichlorohydrine, polyphosphazene, poly(meth)acrylonitrile, an ethylene propylene diene copolymer, polyvinylpyridine, chlorosulfonated polyethylene, latex, a polyester resin, a (meth)acrylic resin, a phenol resin, an epoxy resin, polyvinyl alcohol, and/or a (e.g., any suitable) combination thereof.

If (e.g., when) an aqueous binder is utilized as the negative electrode binder, it may further include a cellulose-based compound capable of imparting viscosity. As this cellulose-based compound, one or more types (kinds) of carboxymethyl cellulose, hydroxypropylmethyl cellulose, methyl cellulose, or an alkali metal salt thereof may be utilized. The alkali metal may be Na, K, or Li.

The dry binder may be a polymer material capable of being fiberized, and may be, for example, polytetrafluoroethylene, polyvinylidene fluoride, a polyvinylidene fluoride-hexafluoropropylene copolymer, polyethylene oxide, and/or a (e.g., any suitable) combination thereof.

The conductive material is utilized to impart conductivity to the electrode, and in the battery being configured, any electronically conductive material can be utilized as long as it does not cause chemical change. Specific examples may include carbon-based materials such as natural graphite, artificial graphite, carbon black, acetylene black, ketjen black, a carbon fiber, a carbon nanofiber, and a carbon nanotube; a metal-based material including copper, nickel, aluminum, silver, and/or the like, in the form of (or provide) a metal powder or a metal fiber; a conductive polymer such as a polyphenylene derivative; and/or a (e.g., any suitable) mixture thereof.

The negative electrode current collector may be of (e.g., may be selected from among) a copper foil, a nickel foil, a stainless steel foil, a titanium foil, a nickel foam, a copper foam, a polymer substrate coated with a conductive metal, and/or a (e.g., any suitable) combination thereof.

### Separator

The rechargeable lithium battery may further include a separator between the negative electrode and the positive electrode, depending on a type or kind of the rechargeable lithium battery. Such a separator may be for example may include polyethylene, polypropylene, polyvinylidene fluoride, and multi-layers thereof such as a polyethylene/polypropylene double-layered separator, a polyethylene/polypropylene/polyethylene triple-layered separator, and a polypropylene/polyethylene/polypropylene triple-layered separator.

The separator may include a porous substrate and a coating layer containing an organic material, an inorganic material, and/or a (e.g., any suitable) combination thereof on a (e.g., one or both surfaces or sides (e.g., opposite surfaces)) of the porous substrate.

The porous substrate may be a polymer film formed of any one selected from among polyolefin such as polyethylene and polypropylene, polyester such as polyethylene terephthalate and polybutylene terephthalate, polyacetal, polyamide, polyimide, polycarbonate, polyether ketone, polyaryl ether ketone, polyetherimide, polyamideimide, polybenzimidazole, polyethersulfone, polyphenylene oxide, a cyclic olefin copolymer, polyphenylene sulfide, polyethylene naphthalate, a glass fiber, Teflon, and polytetrafluoroethylene, or a copolymer and/or a (e.g., any suitable) mixture of two or more of them.

The porous substrate may have a thickness of about 1 $\mu$m to about 40 $\mu$m, for example about 1 $\mu$m to about 30 $\mu$m, about 1 $\mu$m to about 20 $\mu$m, about 5 $\mu$m to about 15 $\mu$m, or about 10 $\mu$m to about 15 $\mu$m.

The organic material may include a (meth)acryl-based copolymer including a first structural unit derived from (meth)acrylamide, and a second structural unit including at least one of a structural unit derived from (meth)acrylic acid or (meth)acrylate, and a structural unit derived from (meth)acrylamidosulfonic acid or a salt thereof.

The inorganic material may include inorganic particles selected from among $Al_2O_3$, $SiO_2$, $TiO_2$, $SnO_2$, $CeO_2$, MgO, NiO, CaO, GaO, ZnO, $ZrO_2$, $Y_2O_3$, $SrTiO_3$, $BaTiO_3$, $Mg(OH)_2$, boehmite, and/or a (e.g., any suitable) combination thereof, but the present disclosure is not limited thereto. An average particle diameter ($D_{50}$) of the inorganic particles may be about 1 nm to about 2000 nm, for example, about 100 nm to about 1000 nm, or about 100 nm to about 700 nm.

The organic material and the inorganic material may be mixed in one coating layer or may exist in a stacked form of (or provide) a coating layer including an organic material and a coating layer including an inorganic material.

A thickness of the coating layer may be each about 0.5 $\mu$m to about 20 $\mu$m, for example, about 1 $\mu$m to about 10 $\mu$m, or about 1 $\mu$m to about 5 $\mu$m.

### Rechargeable Lithium Battery

The rechargeable lithium battery may be classified into cylindrical, prismatic, pouch, coin, and/or the like, depending on their shape. The drawing is a schematic view showing a rechargeable lithium battery according to some example embodiments. Referring to the drawing, the rechargeable lithium battery 100 includes an electrode assembly 40 including a separator 30 interposed between the positive electrode 10 and the negative electrode 20, and a case 50 in which the electrode assembly 40 is housed. The positive electrode 10, the negative electrode 20, and the separator 30 may be impregnated with an electrolyte. The rechargeable lithium battery 100 may include a sealing member 60 that seals the case 50 as shown in the drawing.

Numerical ranges disclosed herein include and are intended to disclose all subsumed sub-ranges of the same numerical precision. For example, a range of "1.0 to 10.0" includes all subranges having a minimum value equal to or greater than 1.0 and a maximum value equal to or less than 10.0, such as, for example, 2.4 to 7.6. Applicant therefore reserves the right to amend this specification, including the claims, to expressly recite any sub-range subsumed within the ranges expressly recited herein.

Hereinafter, examples and comparative examples of the present disclosure will be described. However, the present disclosure is not limited by the following examples.

## EXAMPLES

### Examples 1 to 8 and Comparative Examples 1 to 5

#### (1) Preparation of Electrolyte

As the non-aqueous organic solvent, a carbonate-based solvent mixed in a volume ratio of ethylene carbonate (EC): ethylmethyl carbonate (EMC): dimethyl carbonate (DMC) = 20:10:70 was utilized.

1.5 M lithium salt ($LiPF_6$) was mixed with the non-aqueous organic solvent, and additives were added according to the compositions in Table 1 to obtain electrolytes.

Chemical Formula 1-1-1] (Tris (2,3-epoxypropyl) isocyanurate, CAS No. 2451-62-9

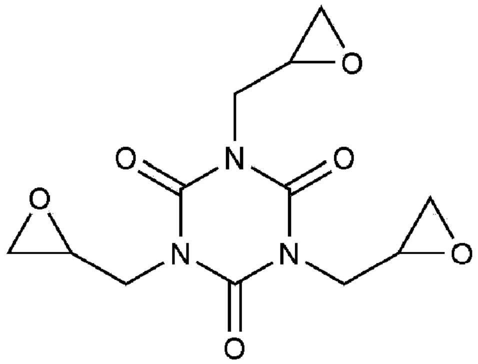

Chemical Formula 1-1-2] (1,3,5-tris[4-(2-oxiranyl)butyl]-1,3,5-triazine-2,4,6-trione, CAS No. 91403-65-5

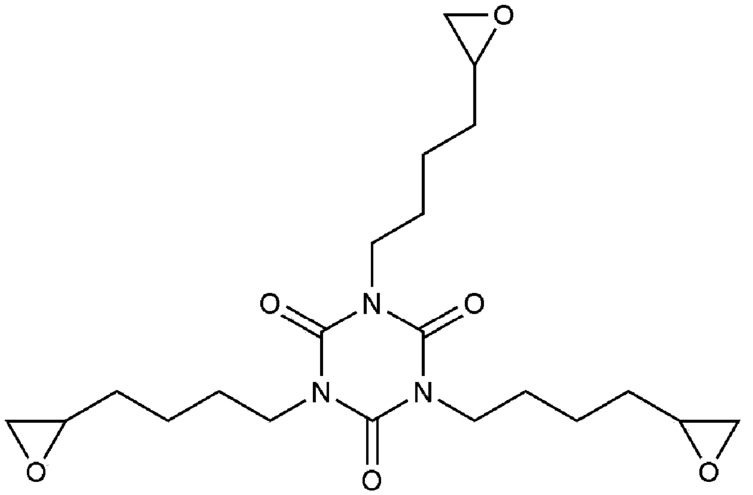

Chemical Formula 2-1a-1] (CAS No. 3965-00-2

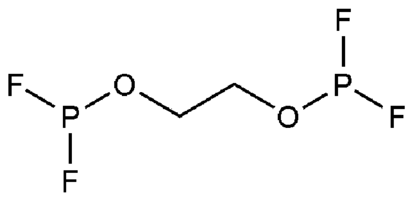

Chemical Formula 2-2a-2] (CAS No. 16415-09-1

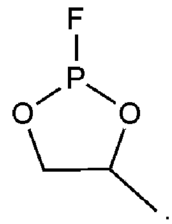

(2) Manufacturing of Rechargeable Lithium Battery Cells

$LiNi_{0.91}Co_{0.04}Al_{0.05}O_2$ as a positive electrode active material, polyvinylidene fluoride as a binder, and ketjen black as a conductive material were mixed respectively in a weight ratio of 98.5:0.75:0.75 and then, dispersed in N-methyl pyrrolidone to prepare a positive electrode active material slurry.

The positive electrode active material slurry was coated on a 14 micrometer (μm)-thick Al foil, dried at 110 °C, and pressed to manufacture a positive electrode.

A mixture including artificial graphite and silicon particles mixed in a weight ratio of 93:7 was utilized as a negative electrode active material, and the negative electrode active material, a styrene-butadiene rubber binder, and carboxylmethyl cellulose in a weight ratio of 97:1:2 were dispersed in distilled water to prepare a negative electrode active material slurry.

The negative electrode active material slurry was coated on a 10 μm-thick Cu foil, dried at 100 °C, and pressed to manufacture a negative electrode.

The manufactured positive and negative electrodes were assembled with a 25 μm-thick polyethylene separator to manufacture an electrode assembly, the electrode assembly was accommodated in a cylindrical can with a width of 21 millimeter (mm) and a length of 70 mm as a battery container, and the electrolyte of the Example 1 was injected thereinto to manufacture a rechargeable lithium battery cell.

Table 1

| | First additive | | Second additive | | Sum content (e.g., Amount) of additives (wt%) |
|---|---|---|---|---|---|
| | Chemical Formula | Content (e.g., Amount ) (wt%) | Chemical Formula | Content (e.g., Amount) (wt%) | |
| Comp. Ex. 1 | - | - | - | - | - |
| Comp. Ex. 2 | Chemical Formula 1-1-1 | 7 | - | - | 7 |
| Comp. Ex. 3 | Chemical Formula 1-1-2 | 7 | - | - | 7 |
| Comp. Ex. 4 | - | - | Chemical Formula 2-1a-1 | 1 | 1 |
| Comp. Ex. 5 | - | - | Chemical Formula 2-2a-2 | 1 | 1 |
| Ex. 1 | Chemical Formula 1-1-1 | 7 | Chemical Formula 2-1a-1 | 1 | 8 |
| Ex. 2 | Chemical Formula 1-1-1 | 7 | Chemical Formula 2-2a-2 | 1 | 8 |
| Ex. 3 | Chemical Formula 1-1-2 | 7 | Chemical Formula 2-1a-1 | 1 | 8 |
| Ex. 4 | Chemical Formula 1-1-2 | 7 | Chemical Formula 2-2a-2 | 1 | 8 |
| Ex. 5 | Chemical Formula 1-1-1 | 5 | Chemical Formula 2-1a-1 | 1 | 6 |
| Ex. 6 | Chemical Formula 1-1-1 | 20 | Chemical Formula 2-1a-1 | 1 | 21 |
| Ex. 7 | Chemical Formula 1-1-1 | 7 | Chemical Formula 2-1a-1 | 0.1 | 7.1 |
| Ex. 8 | Chemical Formula 1-1-1 | 7 | Chemical Formula 2-1a-1 | 2 | 9 |

In the preceding compositions of electrolytes, a "content (e.g., amount) (wt%)" is based on 100 wt% of the total

electrolyte (lithium salt + non-aqueous organic solvent + additives).

**Evaluation Example 1: Evaluation of Heat Exposure Situation**

For each rechargeable lithium battery cell according to Examples 1 to 8 and Comparative Examples 1 to 5, the heat exposure properties were evaluated.

The rechargeable lithium battery cells were heated from room temperature at a rate of 5 °C/min to reach a temperature of 139 °C or higher, and then left to stand at the achieved temperature for 1 hour. The achieved temperature was evaluated as the "heat exposure test passing temperature."

Table 2

| | Heat exposure test passing temperature (°C) |
|---|---|
| Comparative Example 1 | 139 |
| Comparative Example 2 | 142 |
| Comparative Example 3 | 142 |
| Comparative Example 4 | 140 |
| Comparative Example 5 | 140 |
| Example 1 | 143 |
| Example 2 | 143 |
| Example 3 | 143 |
| Example 4 | 143 |
| Example 5 | 143 |
| Example 6 | 143 |
| Example 7 | 143 |
| Example 8 | 143 |

**Evaluation Example 2: Evaluation of High-temperature Storage**

**(1) Capacity Retention Rate and Capacity Recovery Rate**

Each of the rechargeable lithium battery cells of the examples and the comparative examples was once charged and discharged at 0.33 C and measured with respect to charge and discharge capacity (relatively high-temperature storage).

Each of the rechargeable lithium battery cells of the examples and the comparative examples was charged to SOC100% (to reach State of Charge 100% charge capacity based on 100% of total charge capacity of the battery), stored at 60 °C for 30 days, and discharged to 3.0 V at a constant current of 0.33 C and then, measured with respect to initial discharge capacity.

The cells were recharged to 4.3 V at a constant current of 0.33 C and also, to a current of 0.02 C under the constant voltage and then discharged to 3.0 V at 0.33 C to twice measure discharge capacity. A ratio of the first discharge capacity to the initial discharge capacity was shown as a capacity retention rate (retention capacity), and the second discharge capacity was shown as a capacity recovery rate (recovery capacity).

**(2) DC Resistance Change Rate**

Each of the rechargeable lithium battery cells of the examples and the comparative examples was measured with respect to △V/△I (voltage change /current change) to obtain initial DC internal resistance (DCIR) and then, fully charged (SOC 100%) to its internal maximum energy state and stored at a relatively high temperature of 60 °C for 30 days to measure DC resistance, which were utilized to calculate a DCIR increase rate (%) according to Equation 1, and the results are shown in Table 3.

Equation 1

$$\text{DCIR increase rate} = (\text{DCIR after 30 days / initial DCIR}) * 100$$

### (3) Open Circuit Voltage Change Rate

Each of the rechargeable lithium battery cells of the examples and the comparative examples were fully charged to SOC 100% in its internal maximum energy state and stored at a relatively high temperature (55±2 °C) for 90 days. After storing the cells at a relatively high temperature for 90 days, an open circuit voltage (OCV) was calculated according to Equation 2, and the results are shown in Table 3.

Open circuit voltage change rate = (open circuit voltage after x days of relatively high temperature storage/open circuit voltage at the start of storage) Equation 2

Table 3

| | Capacity retention rate (%) | Capacity recovery rate (%) | DC resistance change rate (%) | Open circuit voltage change rate (%) |
|---|---|---|---|---|
| Comparative Example 1 | 81.05 | 85.47 | 153 | -2.04 |
| Comparative Example 2 | 80.54 | 84.33 | 157 | -2.14 |
| Comparative Example 3 | 80.77 | 84.41 | 158 | -2.18 |
| Comparative Example 4 | 85.13 | 86.16 | 146 | -1.87 |
| Comparative Example 5 | 84.93 | 86.17 | 145 | -1.83 |
| Example 1 | 82.39 | 85.91 | 149 | -1.97 |
| Example 2 | 82.13 | 85.78 | 148 | -1.93 |
| Example 3 | 82.07 | 85.66 | 151 | -1.93 |
| Example 4 | 82.09 | 85.76 | 150 | -1.92 |
| Example 5 | 83.94 | 86.05 | 147 | -1.91 |
| Example 6 | 83.46 | 86.09 | 147 | -1.93 |
| Example 7 | 82.97 | 85.94 | 149 | -1.96 |
| Example 8 | 85.64 | 87.17 | 143 | -1.70 |

### Summary

Referring to Table 2, if (e.g., when) either one type or kind of additive out of the two types (kinds) of additives was utilized, the thermal exposure was improved to 142 °C, but if (e.g., when) the two types (kinds) of additives were combined, the thermal exposure was improved to 143 °C, (e.g., by additionally 1°C or more).

In contrast, referring to Table 3, if (e.g., when) the two types (kinds) of additives were combined, a content (e.g., amount) of each additive and a total content (e.g., amount) of the two additives should be limited by comprehensively considering storage characteristics at a relatively high temperature.

For example, based on a total amount of the electrolyte, the first additive should be utilized within a range of 5 to 20 wt%, the second additive should be utilized within a range of 0.1 to 2 wt%, and combined (e.g., all) the additives should be utilized within a range of 7.1 to 21 wt% in total.

### Reference Numerals

100: rechargeable lithium battery
10: positive electrode

20: negative electrode
30: separator
40: electrode assembly
50: case
60: sealing member

## Claims

**1.** An electrolyte for a rechargeable lithium battery (100), comprising:

a non-aqueous organic solvent;
a lithium salt;
a first additive represented by Chemical Formula 1; and
a second additive represented by Chemical Formula 2:

Chemical Formula 1

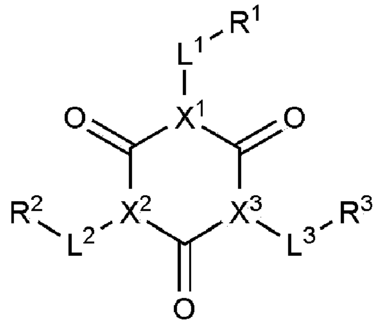

in Chemical Formula 1,
$X^1$ to $X^3$ are each N or CH and at least one selected from among $X^1$ to $X^3$ is N;
$L^1$ to $L^3$ are each independently a single bond, or a substituted or unsubstituted C1 to C10 alkylene group; and
$R^1$ to $R^3$ are each independently an epoxy group, or a substituted or unsubstituted C1 to C10 alkyl group, and at least one selected from among $R^1$ to $R^3$ is an epoxy group;

Chemical Formula 2

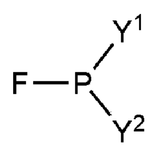

in Chemical Formula 2,
$Y^1$ and $Y^2$ are each independently a halogen or $-O-L^4-R^4$, and at least one selected from among $Y^1$ and $Y^2$ is $-O-L^4-R^4$;
$L^4$ is a single bond or a substituted or unsubstituted C1 to C10 alkylene group;
$R^4$ is a cyano group (-CN), a difluorophosphite group ($-OPF_2$), a substituted or unsubstituted C1 to C10 alkyl group, a substituted or unsubstituted C2 to C10 alkenyl group, a substituted or unsubstituted C3 to C10 cycloalkyl group, a substituted or unsubstituted C3 to C10 cycloalkenyl group, a substituted or unsubstituted C2 to C10 alkynyl group, a substituted or unsubstituted C3 to C10 cycloalkynyl group, or a substituted or unsubstituted C6 to C20 aryl group; and
when $Y^1$ and $Y^2$ are simultaneously $-O-L^4-R^4$, two $R^4$s are not linked or are linked to form a substituted or unsubstituted monocyclic or polycyclic aliphatic heterocycle, or a substituted or unsubstituted monocyclic or polycyclic aromatic heterocycle,
wherein "substituted" refers to replacement of at least one hydrogen by a halogen atom (F, Cl, Br, or I), a hydroxy group, a C1 to C20 alkoxy group, a nitro group, a cyano group, an amine group, an imino group, an azido group, an amidino group, a hydrazino group, a hydrazono group, a carbonyl group, a carbamyl group, a thiol group, an ester

group, an ether group, a carboxyl group or a salt thereof, a sulfonic acid group or a salt thereof, a phosphoric acid or a salt thereof, a C1 to C20 alkyl group, a C2 to C20 alkenyl group, a C2 to C20 alkynyl group, a C6 to C30 aryl group, a C3 to C20 cycloalkyl group, a C3 to C20 cycloalkenyl group, a C3 to C20 cycloalkynyl group, a C2 to C20 heterocycloalkyl group, a C2 to C20 heterocycloalkenyl group, or a C2 to C20 heterocycloalkynyl group.

**2.** The electrolyte as claimed in claim 1, wherein in Chemical Formula 1, $X^1$ to $X^3$ are each N.

**3.** The electrolyte as claimed in claim 1 or claim 2, wherein in Chemical Formula 1, $L^1$ to $L^3$ are each independently a substituted or unsubstituted C1 to C5 alkylene group.

**4.** The electrolyte as claimed in any one of the preceding claims, wherein in Chemical Formula 1, $R^1$ to $R^3$ are each an epoxy group.

**5.** The electrolyte as claimed in claim 1, wherein

the first additive is represented by Chemical Formula 1-1:

Chemical Formula 1-1

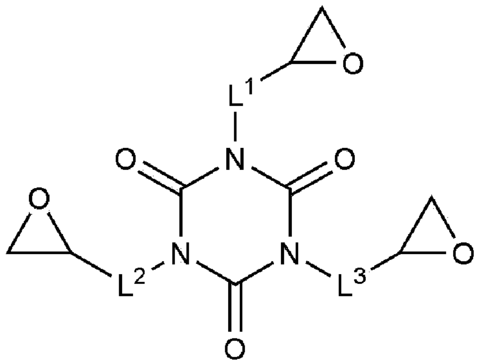

in Chemical Formula 1-1,
$L^1$ to $L^3$ are each independently a substituted or unsubstituted C1 to C5 alkylene group.

**6.** The electrolyte as claimed in claim 1, wherein the first additive is selected from among Chemical Formula 1-1-1 and Chemical Formula 1-1-2:

Chemical Formula 1-1-1

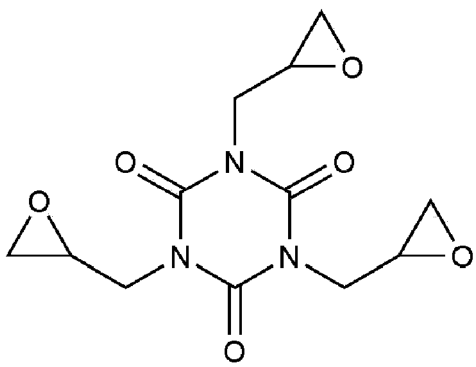

Chemical Formula 1-1-2

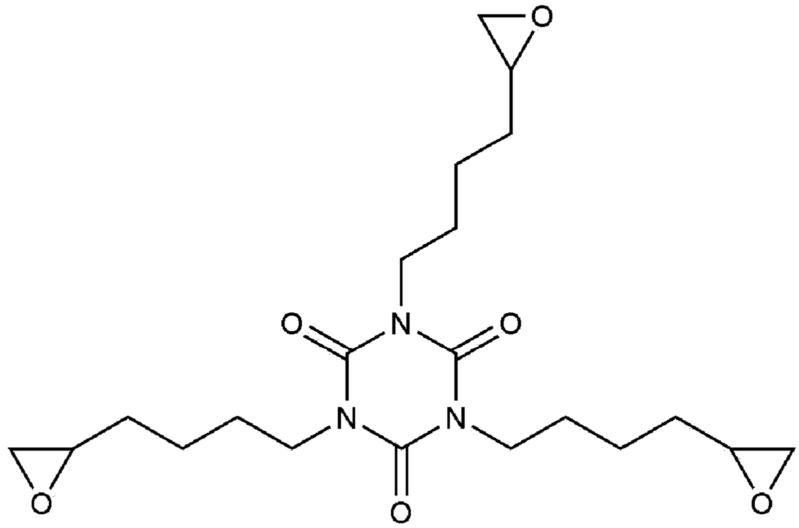

.

7. The electrolyte as claimed in any one of the preceding claims, wherein in Chemical Formula 2:

one selected from among $Y^1$ and $Y^2$ is a fluoro atom and one selected from among $Y^1$ or $Y^2$ is $-O-L^5-R^5$, $L^5$ is a single bond or a substituted or unsubstituted C1 to C10 alkylene group, and $R^5$ is a cyano group (-CN) or a difluorophosphite group ($-OPF_2$); or
$Y^1$ is $-O-L^6-R^6$ and $Y^2$ is $O-L^7-R^7$, $L^6$ and $L^7$ are each independently a single bond or a substituted or unsubstituted C1 to C10 alkylene group, $R^6$ and $R^7$ are each independently a substituted or unsubstituted C1 to C10 alkyl group, and $R^6$ and $R^7$ are linked to form a substituted or unsubstituted monocyclic or polycyclic aliphatic heterocycle.

8. The electrolyte as claimed in claim 7, wherein

the second additive is represented by Chemical Formula 2-1 or Chemical Formula 2-2:

Chemical Formula 2-1

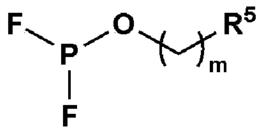

in Chemical Formula 1-1,
m is an integer of 1 to 5; and
$R^5$ is -CN or $-OPF_2$,

Chemical Formula 2-2

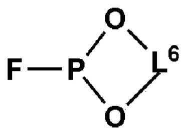

in Chemical Formula 1-2,
$L^6$ is a substituted or unsubstituted C2 to C5 alkylene group.

9. The electrolyte as claimed in claim 8, wherein

the second additive is represented by Chemical Formula 2-2a or Chemical Formula 2-2b:

Chemical Formula 2-2a

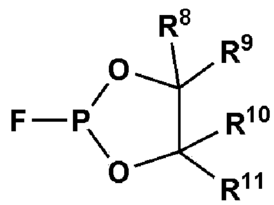

Chemical Formula 2-2b

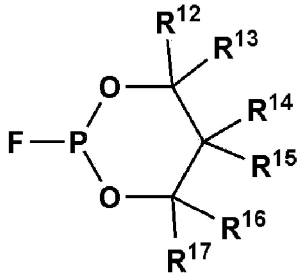

in Chemical Formulas 2-2a and 2-2b,
$R^8$ to $R^{17}$ are each independently a hydrogen atom, a halogen atom, or a substituted or unsubstituted C1 to C5 alkyl group.

**10.** The electrolyte as claimed in any one of claims 1 to 6, wherein the second additive is one selected from among:

Chemical Formula 2-1a-1

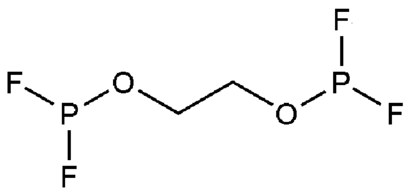

,

Chemical Formula 2-1a-2

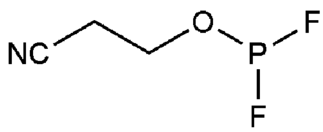

,

Chemical Formula 2-2a-1

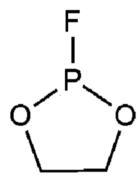

,

and

Chemical Formula 2-2a-2

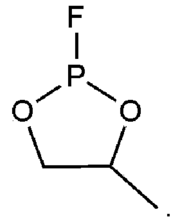

11. The electrolyte as claimed in any one of the preceding claims, wherein a weight ratio of the first additive to the second additive is 2:1 to 100:1.

12. The electrolyte as claimed in any one of the preceding claims, wherein the electrolyte comprises the first additive in an amount of 5 to 20 wt%, based on a total amount of the electrolyte.

13. The electrolyte as claimed in any one of the preceding claims, wherein the electrolyte comprises the second additive in an amount of 0.1 to 2 wt%, based on a total amount of the electrolyte.

14. The electrolyte as claimed in any one of the preceding claims, wherein the non-aqueous organic solvent comprises ethylene carbonate (EC), ethylmethyl carbonate (EMC), and dimethyl carbonate (DMC).

15. A rechargeable lithium battery (100) comprising:

a positive electrode (10) comprising a positive electrode active material;
a negative electrode (20) comprising a negative electrode active material; and
the electrolyte as claimed in claim 1.

**Patentansprüche**

1. Elektrolyt für eine wiederaufladbare Lithiumbatterie (100), umfassend:

ein nicht wässriges organisches Lösungsmittel;
ein Lithiumsalz;
einen ersten Zusatzstoff, dargestellt durch die chemische Formel 1; und
einen zweiten Zusatzstoff, dargestellt durch die chemische Formel 2:

Chemische Formel 1

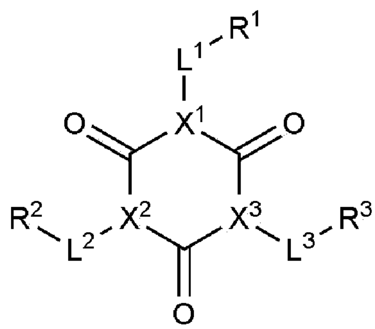

in der chemischen Formel 1
stehen $X^1$ bis $X^3$ jeweils für N oder CH und mindestens eines, das aus $X^1$ bis $X^3$ ausgewählt ist, steht für N;
stehen $L^1$ bis $L^3$ jeweils unabhängig für eine Einfachbindung oder eine substituierte oder unsubstituierte C1- bis

C10-Alkylengruppe; und
stehen $R^1$ bis $R^3$ jeweils unabhängig für eine Epoxidgruppe oder eine substituierte oder unsubstituierte C1- bis C10-Alkylgruppe und mindestens eines, das aus $R^1$ bis $R^3$ ausgewählt ist, steht für eine Epoxidgruppe;

Chemische Formel 2

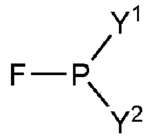

in der chemischen Formel 2
stehen $Y^1$ und $Y^2$ jeweils unabhängig für ein Halogen oder -O-$L^4$-$R^4$ und mindestens eines, das aus $Y^1$ und $Y^2$ ausgewählt ist, steht für -O-$L^4$-$R^4$;
steht $L^4$ für eine Einfachbindung oder eine substituierte oder unsubstituierte C1- bis C10-Alkylengruppe;
steht $R^4$ für eine Cyanogruppe (-CN), eine Difluorphosphitgruppe ($-OPF_2$), eine substituierte oder unsubstituierte C1- bis C10-Alkylgruppe, eine substituierte oder unsubstituierte C2- bis C10-Alkenylgruppe, eine substituierte oder unsubstituierte C3-bis C10-Cycloalkylgruppe, eine substituierte oder unsubstituierte C3- bis C10-Cycloalkenylgruppe, eine substituierte oder unsubstituierte C2- bis C10-Alkinylgruppe, eine substituierte oder unsubstituierte C3- bis C10-Cycloalkinylgruppe oder eine substituierte oder unsubstituierte C6- bis C20-Arylgruppe; und wenn $Y^1$ und $Y^2$ gleichzeitig für -O-$L^4$-$R^4$ stehen, sind zwei $R^4$s nicht verbunden oder so verbunden, dass sie einen substituierten oder unsubstituierten monocyclischen oder polycyclischen aliphatischen Heterocyclus oder einen substituierten oder unsubstituierten monocyclischen oder polycyclischen aromatischen Heterocyclus bilden,
wobei sich "substituiert" auf den Austausch mindestens eines Wasserstoffs durch ein Halogenatom (F, Cl, Br oder I), eine Hydroxygruppe, eine C1- bis C20-Alkoxygruppe, eine Nitrogruppe, eine Cyanogruppe, eine Aminogruppe, eine Iminogruppe, eine Azidogruppe, eine Amidinogruppe, eine Hydrazinogruppe, eine Hydrazonogruppe, eine Carbonylgruppe, eine Carbamylgruppe, eine Thiolgruppe, eine Estergruppe, eine Ethergruppe, eine Carboxylgruppe oder ein Salz davon, eine Sulfonsäuregruppe oder ein Salz davon, eine Phosphorsäuregruppe oder ein Salz davon, eine C1- bis C20-Alkylgruppe, eine C2- bis C20-Alkenylgruppe, eine C2- bis C20-Alkinylgruppe, eine C6-bis C30-Arylgruppe, eine C3- bis C20-Cycloalkylgruppe, eine C3- bis C20-Cycloalkenylgruppe, eine C3- bis C20-Cycloalkinylgruppe, eine C2- bis C20-Heterocycloalkylgruppe, eine C2- bis C20-Heterocycloalkenylgruppe oder eine C2- bis C20-Heterocycloalkinylgruppe bezieht.

**2.** Elektrolyt nach Anspruch 1, wobei
in der chemischen Formel 1 $X^1$ bis $X^3$ jeweils für N stehen.

**3.** Elektrolyt nach Anspruch 1 oder Anspruch 2, wobei
in der chemischen Formel 1 $L^1$ bis $L^3$ jeweils unabhängig für eine substituierte oder unsubstituierte C1- bis C5-Alkylengruppe stehen.

**4.** Elektrolyt nach einem der vorhergehenden Ansprüche, wobei in der chemischen Formel 1 $R^1$ bis $R^3$ jeweils für eine Epoxidgruppe stehen.

**5.** Elektrolyt nach Anspruch 1, wobei

der erste Zusatzstoff durch die chemische Formel 1-1 dargestellt ist:

Chemische Formel 1-1

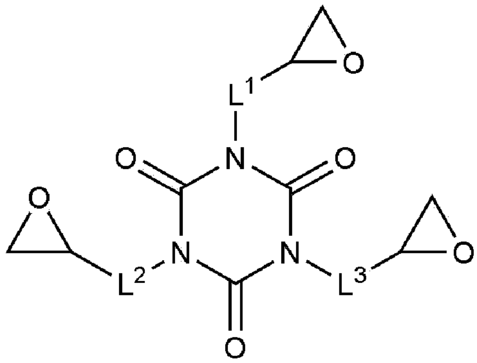

in der chemischen Formel 1-1
$L^1$ bis $L^3$ jeweils unabhängig für eine substituierte oder unsubstituierte C1- bis C5-Alkylengruppe stehen.

**6.** Elektrolyt nach Anspruch 1, wobei der erste Zusatzstoff ausgewählt ist aus der chemischen Formel 1-1-1 und der chemischen Formel 1-1-2:

Chemische Formel 1-1-1

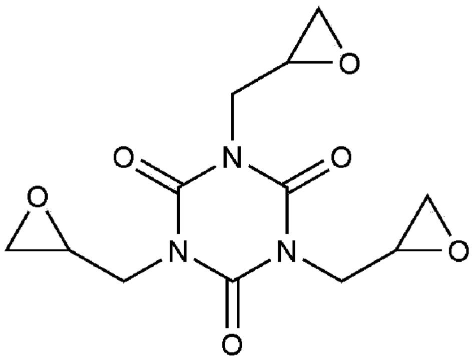

Chemische Formel 1-1-2

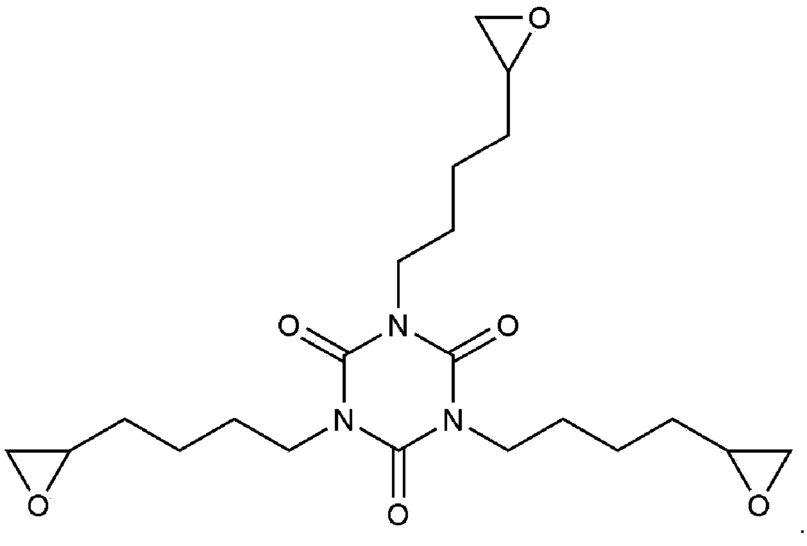

**7.** Elektrolyt nach einem der vorhergehenden Ansprüche, wobei in der chemischen Formel 2:

eines, das aus $Y^1$ und $Y^2$ ausgewählt ist, für ein Fluoratom steht und eines, das aus $Y^1$ oder $Y^2$ ausgewählt ist, für -O-$L^5$-$R^5$ steht, $L^5$ für eine Einfachbindung oder eine substituierte oder unsubstituierte C1- bis C10-Alkylengruppe steht und $R^5$ für eine Cyanogruppe (-CN) oder eine Difluorphosphitgruppe (-$OPF_2$) steht; oder
$Y^1$ für -O-$L^6$-$R^6$ steht und $Y^2$ für O-$L^7$-$R^7$ steht, $L^6$ und $L^7$ jeweils unabhängig für eine Einfachbindung oder eine substituierte oder unsubstituierte C1- bis C10-Alkylengruppe stehen, $R^6$ und $R^7$ jeweils unabhängig für eine substituierte oder unsubstituierte C1- bis C10-Alkylgruppe stehen und $R^6$ und $R^7$ so verbunden sind, dass sie einen substituierten oder unsubstituierten monocyclischen oder polycyclischen aliphatischen Heterocyclus bilden.

**8.** Elektrolyt nach Anspruch 7, wobei

der zweite Zusatzstoff durch die chemische Formel 2-1 oder die chemische Formel 2-2 dargestellt ist:

Chemische Formel 2-1

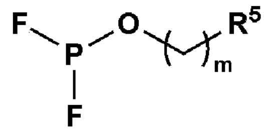

in der chemischen Formel 1-1
m für eine ganze Zahl von 1 bis 5 steht; und
$R^5$ für -CN oder -$OPF_2$ steht,

Chemische Formel 2-2

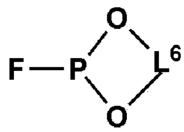

in der chemischen Formel 1-2
$L^6$ für eine substituierte oder unsubstituierte C2- bis C5-Alkylengruppe steht.

**9.** Elektrolyt nach Anspruch 8, wobei

der zweite Zusatzstoff durch die chemische Formel 2-2a oder die chemische Formel 2-2b dargestellt ist:

Chemische Formel 2-2a

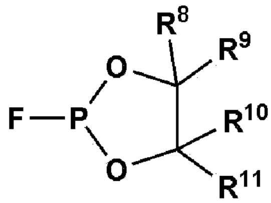

Chemische Formel 2-2b

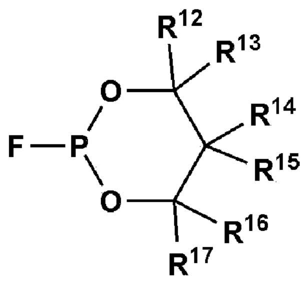

in den chemischen Formeln 2-2a und 2-2b
$R^8$ bis $R^{17}$ jeweils unabhängig für ein Wasserstoffatom, ein Halogenatom oder eine substituierte oder unsubstituierte C1- bis C5-Alkylgruppe stehen.

**10.** Elektrolyt nach einem der Ansprüche 1 bis 6, wobei der zweite Zusatzstoff einer ist, der ausgewählt ist aus:

der chemischen Formel 2-1a-1

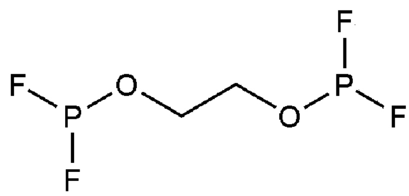

,

der chemischen Formel 2-1a-2

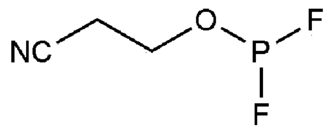

,

der chemischen Formel 2-2a-1

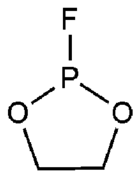

,

und

der chemischen Formel 2-2a-2

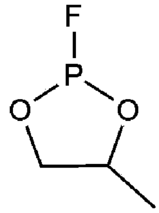

.

**11.** Elektrolyt nach einem der vorhergehenden Ansprüche, wobei ein Gewichtsverhältnis des ersten Zusatzstoffs zum

zweiten Zusatzstoff 2:1 bis 100:1 beträgt.

**12.** Elektrolyt nach einem der vorhergehenden Ansprüche, wobei der Elektrolyt den ersten Zusatzstoff in einer Menge von 5 bis 20 Gew.-% basierend auf einer Gesamtmenge des Elektrolyten umfasst.

**13.** Elektrolyt nach einem der vorhergehenden Ansprüche, wobei der Elektrolyt den zweiten Zusatzstoff in einer Menge von 0,1 bis 2 Gew.-% basierend auf einer Gesamtmenge des Elektrolyten umfasst.

**14.** Elektrolyt nach einem der vorhergehenden Ansprüche, wobei das nicht wässrige organische Lösungsmittel Ethylencarbonat (EC), Ethylmethylcarbonat (EMC) und Dimethylcarbonat (DMC) umfasst.

**15.** Wiederaufladbare Lithiumbatterie (100), umfassend:

eine positive Elektrode (10), die ein Aktivmaterial für die positive Elektrode umfasst;
eine negative Elektrode (20), die ein Aktivmaterial für die negative Elektrode umfasst; und
den Elektrolyten nach Anspruch 1.

**Revendications**

**1.** Électrolyte pour une batterie rechargeable au lithium (100), comportant :

un solvant organique non aqueux ;
un sel de lithium ;
un premier additif représenté par la Formule chimique 1 ; et
un deuxième additif représenté par la Formule chimique 2 :

Formule chimique 1

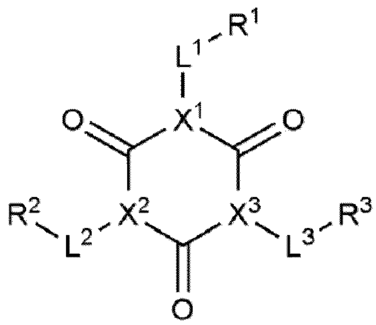

dans la Formule chimique 1,
$X^1$ à $X^3$ sont chacun N ou CH et au moins l'un choisi parmi $X^1$ à $X^3$ est N ;
$L^1$ à $L^3$ sont chacun indépendamment une liaison unique, ou un groupe alkylène en C1 à C10 substitué ou non substitué ; et
$R^1$ à $R^3$ sont chacun indépendamment un groupe époxy, ou un groupe alkyle en C1 à C10 substitué ou non substitué, et au moins l'un choisi parmi $R^1$ à $R^3$ est un groupe époxy ;

Formule chimique 2

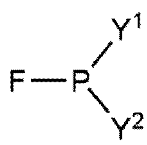

dans la Formule chimique 2,
$Y^1$ et $Y^2$ sont chacun indépendamment un halogène ou -O-$L^4$-$R^4$, et au moins l'un choisi parmi $Y^1$ et $Y^2$ est -O-

$L^4$-$R^4$ ;
$L^4$ est une liaison unique ou un groupe alkylène en C1 à C10 substitué ou non substitué ;
$R^4$ est un groupe cyano (-CN), un groupe difluorophosphite (-$OPF_2$), un groupe alkyle en C1 à C10 substitué ou non substitué, un groupe alcényle en C2 à C10 substitué ou non substitué, un groupe cycloalkyle en C3 à C10 substitué ou non substitué, un groupe cycloalcényle en C3 à C10 substitué ou non substitué, un groupe alcynyle en C2 à C10 substitué ou non substitué, ou un groupe cycloalcynyle en C3 à C10 substitué ou non substitué, ou un groupe aryle en C6 à C20 substitué ou non substitué ; et
lorsque $Y^1$ et $Y^2$ sont simultanément -O-$L^4$-$R^4$, deux $R^4$ ne sont pas liés ou sont liés pour former un hétérocycle aliphatique monocyclique ou polycyclique substitué ou non substitué, ou un hétérocycle aromatique monocyclique ou polycyclique substitué ou non substitué,
dans lequel « substitué » fait référence au remplacement d'au moins un hydrogène par un atome d'halogène (F, Cl, Br ou 1), un groupe hydroxy, un groupe alcoxy en C1 à C20, un groupe nitro, un groupe cyano, un groupe amine, un groupe imino, un groupe azido, un groupe amidino, un groupe hydrazino, un groupe hydrazono, un groupe carbonyle, un groupe carbamyle, un groupe thiol, un groupe ester, un groupe éther, un groupe carboxyle ou un sel de celui-ci, un groupe acide sulfonique ou un sel de celui-ci, un acide phosphorique ou un sel de celui-ci, un groupe alkyle en C1 à C20, un groupe alcényle en C2 à C20, un groupe alcynyle en C2 à C20, un groupe aryle en C6 à C30, un groupe cycloalkyle en C3 à C20, un groupe cycloalcényle en C3 à C20, un groupe cycloalcynyle en C3 à C20, un groupe hétérocycloalkyle en C2 à C20, un groupe hétérocycloalcényle en C2 à C20 ou un groupe hétérocycloalcynyle en C2 à C20.

**2.** Électrolyte selon la revendication 1, dans lequel
dans la Formule chimique 1, $X^1$ à $X^3$ sont chacun N.

**3.** Électrolyte selon la revendication 1 ou la revendication 2, dans lequel
dans la Formule chimique 1, $L^1$ à $L^3$ sont chacun indépendamment un groupe alkylène en C1 à C5 substitué ou non substitué.

**4.** Électrolyte selon l'une quelconque des revendications précédentes, dans lequel
dans la Formule chimique 1, $R^1$ à $R^3$ sont chacun un groupe époxy.

**5.** Électrolyte selon la revendication 1, dans lequel

le premier additif est représenté par la Formule chimique 1-1 :

Formule chimique 1-1

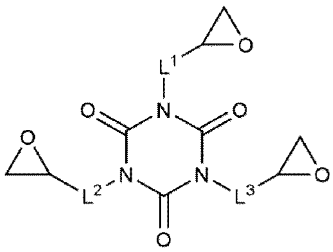

dans la Formule chimique 1-1,
$L^1$ à $L^3$ sont chacun indépendamment un groupe alkylène en C1 à C5 substitué ou non substitué.

**6.** Électrolyte selon la revendication 1, dans lequel
le premier additif est choisi parmi la Formule chimique 1-1-1 et la Formule chimique 1-1-2 :

Formule chimique 1-1-1

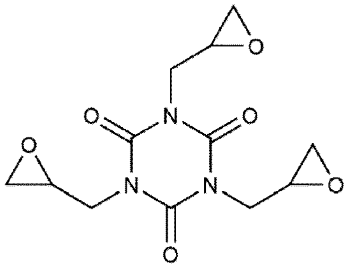

Formule chimique 1-1-2

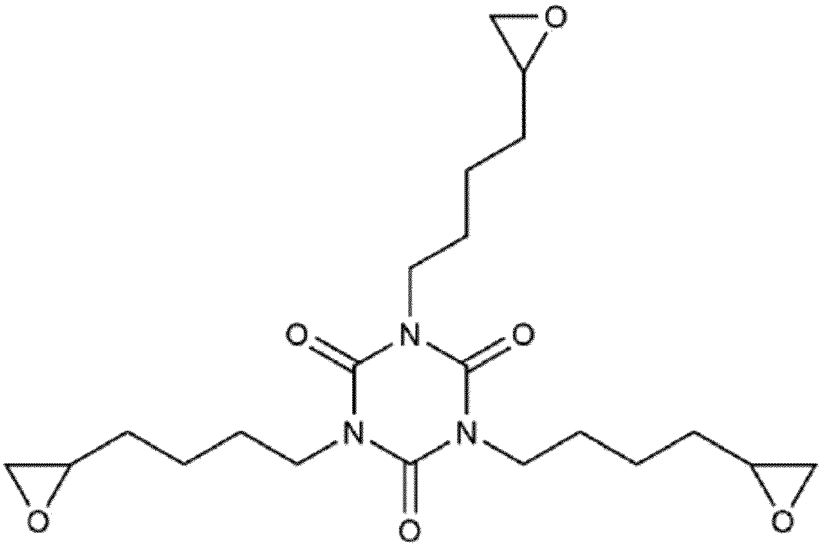

7. Électrolyte selon l'une quelconque des revendications précédentes, dans lequel, dans la Formule chimique 2 :

l'un choisi parmi $Y^1$ et $Y^2$ est un atome fluoré et l'un choisi parmi $Y^1$ ou $Y^2$ est -O-$L^5$-$R^5$, $L^5$ est une liaison unique ou un groupe alkylène en C1 à C10 substitué ou non substitué, et $R^5$ est un groupe cyano (-CN) ou un groupe difluorophosphite (-$OPF_2$) ; ou
$Y^1$ est -O-$L^6$-$R^6$ et $Y^2$ est O-$L^7$-$R^7$, $L^6$ et $L^7$ sont chacun indépendamment une liaison unique ou un groupe alkylène en C1 à C10 substitué ou non substitué, $R^6$ et $R^7$ sont chacun indépendamment un groupe alkyle en C1 à C10 substitué ou non substitué, et $R^6$ et $R^7$ sont liés pour former un hétérocycle aliphatique monocyclique ou polycyclique substitué ou non substitué.

8. Électrolyte selon la revendication 7, dans lequel

le deuxième additif est représenté par la Formule chimique 2-1 ou la Formule chimique 2-2 :

Formule chimique 2-1

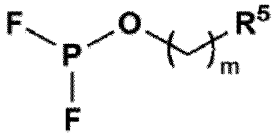

dans la Formule chimique 1-1,
m est un nombre entier de 1 à 5 ; et
$R^5$ est -CN ou -$OPF_2$,

Formule chimique 2-2

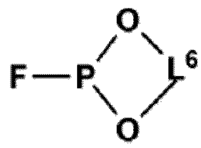

dans la Formule chimique 1-2
$L^6$ est un groupe alkylène en C2 à C5 substitué ou non substitué.

**9.** Électrolyte selon la revendication 8, dans lequel

le deuxième additif est représenté par la Formule chimique 2-2a ou la Formule chimique 2-2b :

Formule chimique 2-2a

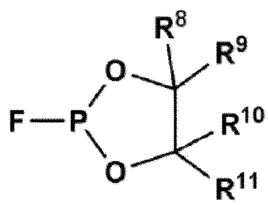

Formule chimique 2-2b

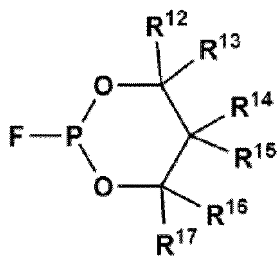

dans les Formules chimiques 2-2a et 2-2b,
$R^8$ à $R^{17}$ sont chacun indépendamment un atome d'hydrogène, un atome d'halogène ou un groupe alkyle en C1 à C5 substitué ou non substitué.

**10.** Électrolyte selon l'une quelconque des revendications 1 à 6, dans lequel le deuxième additif est l'un choisi parmi :

Formule chimique 2-1a-1

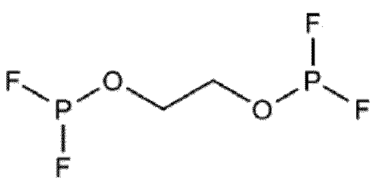

,

Formule chimique 2-1a-2

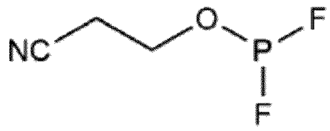
,

Formule chimique 2-2a-1

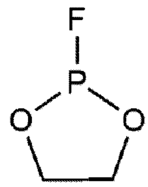

et

Formule chimique 2-2a-2

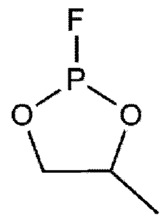
.

**11.** Électrolyte selon l'une quelconque des revendications précédentes, dans lequel un rapport pondéral entre le premier additif et le deuxième additif est de 2:1 à 100:1.

**12.** Électrolyte selon l'une quelconque des revendications précédentes, dans lequel l'électrolyte comporte le premier additif en une quantité de 5 à 20 % en poids, par rapport à une quantité totale de l'électrolyte.

**13.** Électrolyte selon l'une quelconque des revendications précédentes, dans lequel l'électrolyte comporte le deuxième additif en une quantité de 0,1 à 2 % en poids, par rapport à une quantité totale de l'électrolyte.

**14.** Électrolyte selon l'une quelconque des revendications précédentes, dans lequel le solvant organique non aqueux comporte du carbonate d'éthylène (CE), du carbonate d'éthylméthyle (CEM) et du carbonate de diméthyle (DMC).

**15.** Batterie rechargeable au lithium (100) comportant :

une électrode positive (10) comportant un matériau actif d'électrode positive ;
une électrode négative (20) comportant un matériau actif d'électrode négative ; et l'électrolyte selon la revendication 1.

**FIG.1**

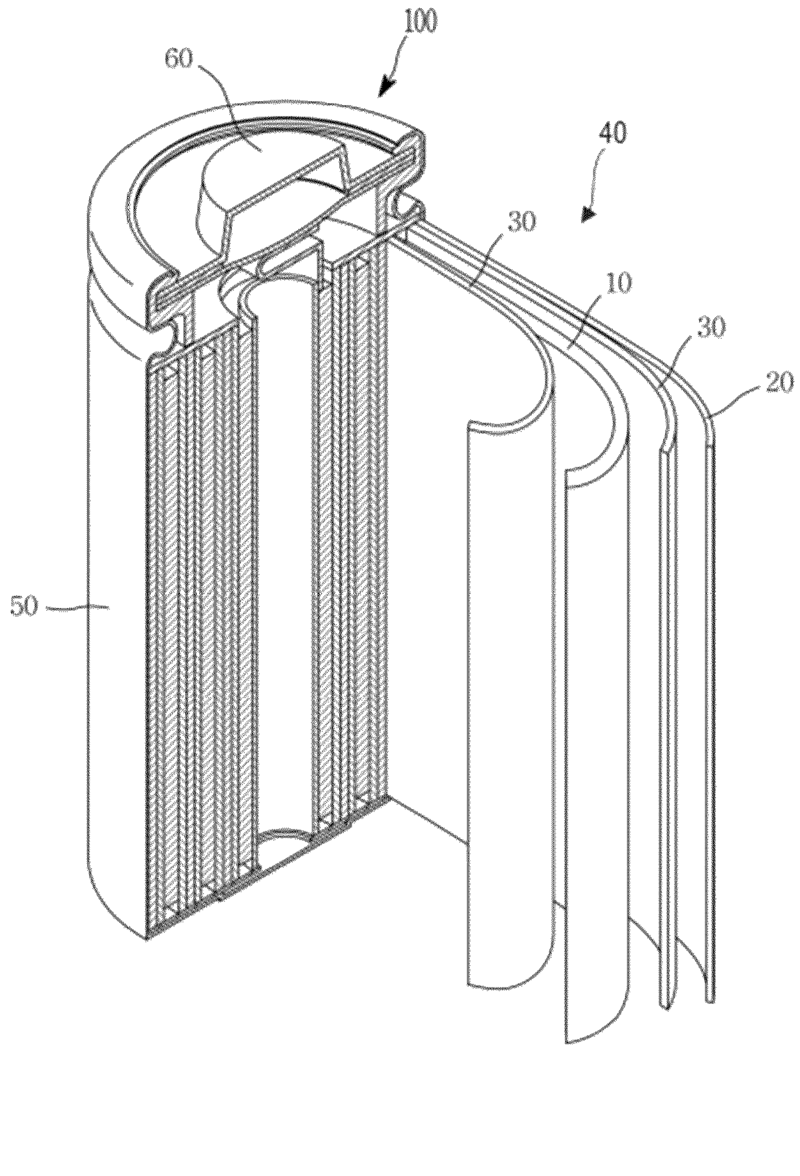

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014308564 A1
- US 2012088160 A1
- WO 2022150849 A1
- JP 2017228520 A